(19) Europäisches Patentamt
European Patent Office
Office européen des brevets

(11) Publication number: 0 113 213
B1

(12) # EUROPEAN PATENT SPECIFICATION

(45) Date of publication of patent specification: 06.04.88

(21) Application number: 83307534.4

(22) Date of filing: 12.12.83

(51) Int. Cl.[4]: C 07 D 231/06,
C 07 D 405/12, A 01 N 43/56
// C07C147/00, C07C49/00

(54) **Pyrazoline insecticides.**

(30) Priority: 30.12.82 GB 8236976

(43) Date of publication of application:
11.07.84 Bulletin 84/28

(45) Publication of the grant of the patent:
06.04.88 Bulletin 88/14

(84) Designated Contracting States:
BE CH DE FR GB IT LI LU NL SE

(56) References cited:
EP-A-0 004 733
EP-A-0 058 424

(73) Proprietor: SCHERING AGROCHEMICALS LIMITED
Hauxton Cambridge CB2 5HU (GB)

(72) Inventor: Giles, David Peter
9 Brookhampton Street
Ickleton Cambridgeshire (GB)
Inventor: Willis, Robert John
35 The Croft
Fulbourn Cambridgeshire (GB)

(74) Representative: Waldman, Ralph David et al
Schering Agrochemicals Limited
Industrial Property Department
Chesterford Park Research Station
Saffron Walden Essex CB10 1XL (GB)

EP 0 113 213 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to new compounds having insecticidal activity.

Various pyrazoline derivatives have been disclosed as having insecticidal activity. The closes prior art to the compounds of the present invention is described in European Patent Specification No. 58424 which claims compounds having the formula

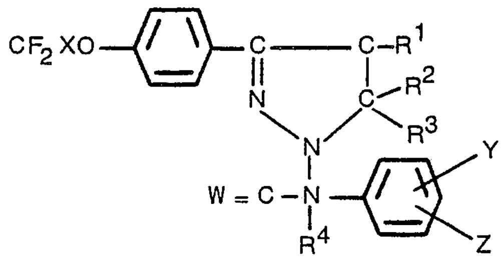

wherein $R^1$ represents hydrogen, a lower alkyl group, a phenyl group, a halogen-substituted phenyl group, a cyanoalkyl group or a lower alkoxyalkyl group: $R^2$ and $R^3$ respectively represent hydrogen, a lower alkyl group, a phenyl group or a halogen-substituted phenyl group; $R^4$ represents hydrogen or a lower alkyl group; X represents hydrogen or halogen; W represents oxygen or sulphur; Y and Z respectively represent hydrogen, a halogen, a lower alkyl group, a lower alkoxy group, a nitro group, a trifluoromethyl group, a lower alkylthio group, an acyl group, a nitrile group, a lower alkylsulphonyl group, a lower alkoxycarbonyl group, —A—$R^5$ or Y and Z form —O—$CH_2$—O—; and A represents —O—, —S—, —SO— or —$SO_2$— and $R^5$ represents a halogen-substituted lower alkyl group.

The compounds of the present invention often have advantages over compounds of the prior art and in particular where we have compared compounds of the present invention over their isomers in the above patent specification we have found unexpected improvements in certain activities. We are also not aware of any pyrazoline compounds of this type which contain phenyl groups on the 3,4 or 5 position which carry a sulphonyloxy group.

Thus according to the invention there are provided compounds of formula I

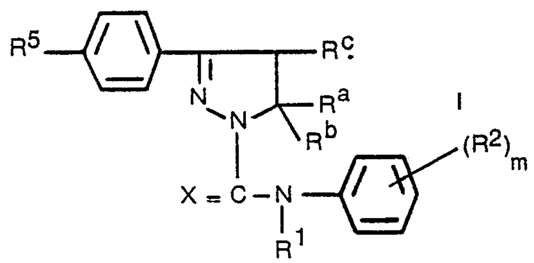

I

where $R^1$ is hydrogen, $C_{1-4}$-alkyl or allyl;

X is oxygen or sulphur;

each $R^2$, which may be the same or different, is halogen, $C_{1-4}$-alkyl, halo-$C_{1-4}$-alkyl, cyano, nitro, $C_{1-4}$-alkoxy, halo-$C_{1-4}$-alkoxy, $C_{1-4}$-alkylthio, halo-$C_{1-4}$-alkylthio, $C_{1-4}$-alkylsulphonyloxy, $C_{1-4}$-alkylsulphonyl, $C_{1-4}$-alkoxycarbonyl, or $C_{1-4}$-alkanoyl or two adjacent $R^2$ groups together with the carbons to which they are attached form a 5 to 7 membered ring, which may comprise one or more oxygen atoms; m is 0 to 4; either

a) $R^a$ is hydrogen and one of $R^b$ and $R^c$ is phenyl, para substituted by the group $R^4$, and the other is hydrogen; or

b) $R^a$ and $R^b$ are both $C_{1-4}$-alkyl and $R^c$ is phenyl para substituted by the group $R^4$; where $R^4$ is halo-$C_{1-4}$-alkoxy;

$R^5$ is $R^8SO_2O$, hydrogen, halogen, $C_{1-4}$-alkyl, halo-$C_{1-4}$-alkoxy, $C_{1-4}$-alkylthio or $C_{1-4}$-alkylsulphonyl, wherein $R^8$ is $C_{1-4}$-alkyl or halo-$C_{1-4}$-alkyl.

It is particularly preferred that $R^4$ is difluoromethoxy. Particularly preferred groups for $R^8$ are methyl and trifluoromethyl. Preferably $R^a$ and $R^b$ are hydrogen. A particularly preferred group of compounds are these in which $R^5$ is hydrogen or halogen, especially chlorine or fluorine. Generally m is 1 and $R^2$ is in the para position. Preferred $R^2$ groups are chlorine, fluorine, bromine or methylthio and especially trifluoromethyl.

The compounds of the invention have insecticidal activity and are particularly useful in combating a variety of economically important insects, e.g. Lepidoptera, including larvae of *Spodoptera littoralis, Heliothis* spp (e.g. *Heliothis armiger), Plutella xylostella, Pieris brassicae,* grape berry moths, eg. *Eupoecilia ambiguella)* and loopers (e.g. *Trichoplusia ni)* Diptera, including *Musca domestica, Ceratatis capitata* and

*Aedes aegypti;* Homoptera, including hoppers e.g. *Niliparvata lugens;* Coleoptera, including *Phaedon cochleariae* and the boll weevil *(Anthonomus grandis);* Orthoptera and Dictyoptera, including *Blattella germanica* and Hymenoptera, especially ants, such as *Solenopsis* spp., *Atta* spp and *Monomorium* spp. The compounds also have activity against human and animal ectoparasites, such as blowfly (*Lucilia* spp,) and lice e.g. *Damalinia bovis.*

The invention also includes an insecticidal composition which comprises a compound of formula I in association with an agronomically acceptable diluent or carrier. More than one compound of the invention can, of course, be included in the composition.

In addition the composition can comprise one or more additional pesticides, for example compounds known to possess herbicidal, fungicidal, insecticidal, acaricidal or nematicidal properties. Alternatively the compounds of the invention can be used in sequence with the other pesticides. Insecticides and acaricides which can be used in conjunction with the compounds of the invention include natural and synthetic pyrethroids (such as the natural pyrethrins, allethrin, bioallethrin, resmethrin, bioresmethrin, tetramethrin, furamethrin, fenpyrithrin, permethrin, cypermethrin, deltamethrin, fenvalerate, alphamethrin, phenothrin, fenpropathrin, flumethrin, empenthrin, prallethrin, tralocythrin, tralomethrin, flucythrinate, fluvalinate, cyfluthrin and cyhalothrin), organophosphorus compounds (such as tetrachlorvinphos, fenitrothion, malathion, dialifos, chlorfenvinphos, dioxathion, demeton-S-methyl, phosalone, dichlorovos, bromophos-ethyl, diazinon, dimethoate, sulprofos, acephate, cylorpyrifos, crufomate, heptenophos, naled, phenthoate, phorate, terbufos, pirimiphos-ethyl, pirimiphos-methyl, parathion-methyl, temephos, famphur, chlormephos, coumaphos, fenthion and phosmet), carbamates (such as bendiocarb, carbofuran, aldicarb, carbaryl, pirimicarb, promecarb, propoxur, formetanate, methomyl, oxamyl, thiofanox, bufencarb, thiodicarb, carbosulfan and dimetilan), chlorinated compounds, (such as toxaphene, endosulphan, HCH and DDT) and miscellaneous compounds including amitraz, chlormethiuron, endosulphan, cyhexatin, diflubenzuron, chlordimeform, benzoximate, dicofol, propargite and clofentezine.

The diluent or carrier in the composition of the invention can be a solid or a liquid optionally in association with a surface-active agent, for example a dispersing agent, emulsifying agent or wettng agent. Suitable surface-active agents include anionic compounds such as a carboxylate, for example a metal carboxylate of a long chain fatty acid; an N-acylsarcosinate; mono- or di-esters or phosphoric acid with fatty alcohol ethoxylates or salts of such esters; fatty alcohol sulphates such as sodium dodecyl sulphate, sodium octadecyl sulphate or sodium cetyl sulphate; ethoxylated fatty alcohol sulphates; ethoxylated alkylphenol sulphates; lignin sulphonates; petroleum sulphonates; alkyl-aryl sulphonates such as alkyl-benzene sulphonates or lower alkylnaphthalene sulphonates, e.g. butyl-naphthalene sulphonate; salts of sulphonated naphthalene-formaldehyde condensates; salts of sulphonated phenol-formaldehyde condensates; or more complex sulphonates such as the amide sulphonates, e.g. the sulphonated condensation product of oleic acid and N-methyl taurine or the dialkyl sulphosuccinates, e.g. the sodium sulphonate of dioctyl succinate. Nonionic agents include condensation products of fatty acid esters, fatty alcohols, fatty acid amides or fatty-alkyl- or alkenyl-substituted phenols with ethylene oxide, fatty esters of polyhydric alcohol ethers, e.g. sorbitan fatty acid esters, condensation products of such esters with ethylene oxide, e.g. polyoxyethylene sorbitan fatty acid esters, block copolymers of ethylene oxide and propylene oxide, acetylenic glycols such as 2,4,7,9-tetramethyl-5-decyn-4,7-diol, or ethoxylated acetylenic glycols. Examples of a cationic surface-active agent include, for instance, an aliphatic mono-, di-, or polyamine as an acetate, naphthenate or oleate; an oxygen-containing amine such as amine oxide or polyoxyethylene alkylamine; an amide-linked amine prepared by the condensation of a carboxylic acid with a di- or polyamine; or a quaternary ammonium salt.

The compositions of the invention can take any form known in the art for the formulation of insecticidal compounds, for example, a solution, a dispersion, an aqueous emulsion, a dusting powder, a seed dressing, a fumigant, a smoke, a dispersible powder, an emulsifiable concentrate, granules or baits. Moreover it can be in a suitable form for direct application or as a concentrate or primary composition which requires dilution with a suitable quantity of water or other diluent before application.

As a dispersion, the composition comprises a compound of the invention dispersed in a liquid medium, preferably water. It is often convenient to supply the consumer with a primary composition which can be diluted with water to form a dispersion having the desired concentration. The primary composition can be provided in any one of the following forms.

An emulsifiable concentrate comprises a compound of the invention dissolved in a water-immiscible solvent together with an emulsifying agent and which is formed into an emulsion on mixing with water. A dusting powder comprises a compound of the invention intimately mixed with a solid pulverulent diluent, for example, kaolin.

A granular solid comprises a compound of the invention associated with similar diluents to those which may be employed in dusting powders, but the mixture is granulated by known methods. Alternatively it comprises the active ingredient adsorbed or absorbed on a pre-granular diluent, for example, Fuller's earth, attapulgite or limestone grit.

A wettable powder usually comprises the active ingredient in admixture with a suitable surfactant and an inert powder diluent such as china clay.

Another suitable concentrate, particularly when the product is a solid, is a flowable suspension concentrate which is formed by grinding the compound with water or an oil, a wetting agent and a

suspending agent.

Baits can include an attractant and may comprise a protein hydrolysate e.g. for the control of fruit flies, sugar e.g. for the control of adult *Musca* spp. or peanut butter or corn cob e.g. for the control of cockroaches or ants.

The concentration of the active ingredient in the composition of the present invention is preferably within the range of 1 to 30 per cent by weight, especially 5 to 30 per cent by weight. In a primary composition the amount of active ingredient can vary widely and can be, for example, from 5 to 95 per cent by weight of the composition.

The compounds of the invention may be prepared by reacting a compound of formula II

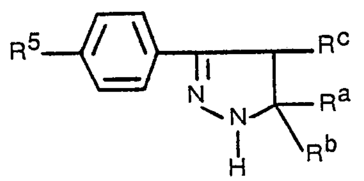

II

(i) with phosgene or thiophosgene followed by a compound of formula

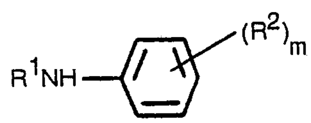

(ii) with a compound of formula

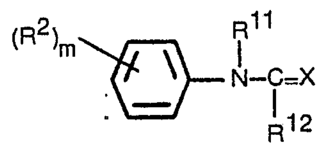

where $R^{11}$ and $R^{12}$ form a double bond when $R^1$ is hydrogen, or $R^{11}$ is $R^1$ and $R^{12}$ is chlorine.

Alternatively a compound of formula III

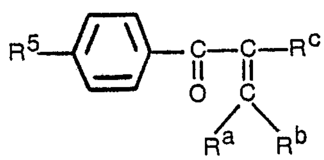

III

can be reacted with a compound of formula IV

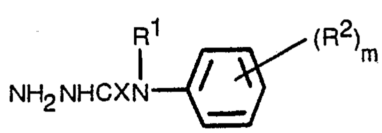

IV

Compounds where $R^1$ is hydrogen can be subsequently alkylated or alkenylated, e.g. by reaction with an alkyl or allyl halide, under basic conditions.

The above reactions may suitably be carried out at a temperature of 0—100°C.

The compounds of formula II are novel and form part of the invention. They may be prepared by reacting a compound of formula III with hydrazine. The compounds of formula III in which $R^c$ is substituted phenyl may be prepared by reacting a compound of formula V

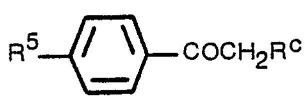

V

with a compound of formula $R^aR^bCO$, e.g. formaldehyde. Compounds in which $R^a$ and $R^b$ are $C_{1-4}$-alkyl are usually prepared by reaction of V with a compound $R^aR^bCHI$, followed by halogenation e.g. with bromine, and dehydrohalogenation, e.g. using lithium chloride.

Compounds of formula V may be prepared from the corresponding hydroxy compound, by reaction

with a polyhaloalkane. Those compounds in which $R^b$ is substituted phenyl may be prepared by reacting a compound of formula

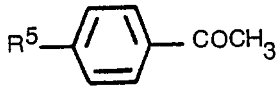

with a compound of formula

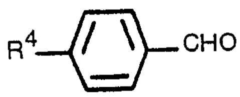

Compounds which comprise a $C_{1-4}$-alkylsulphonyl group may be obtained by oxidising the corresponding compound comprising a $C_{1-4}$-alkylthio group, using a suitable oxidising agent such as hydrogen peroxide.

The invention is illustrated in the following Examples. Structures of isolated novel compounds were confirmed by elemental and/or other appropriate analyses.

Example 1

Chlorodifluoromethane was bubbled into a mixture of 1-(4-chlorophenyl)-2-(4-hydroxyphenyl)ethanone with aqueous sodium hydroxide and dioxan, at elevated temperature for 3 hours. The mixture was then heated for a further period and then kept overnight at room temperature, after which is was poured into water and after a conventional work-up procedure, there was obtained 1-(4-chlorophenyl)-2-(4-difluoromethoxyphenyl)ethanone, m.p. 71—73°C (Intermediate A). This product (26 g) was then treated with aqueous formaldehyde (37% w/v, 24 ml), piperidine (0.9 ml) and glacial acetic acid (0.9 ml) and then heated and worked up in conventional manner to give crude 1-(4-chlorophenyl)-2-(4-difluoromethoxyphenyl)-2-propen-1-one, (Intermediate B). This was then treated with hydrazine hydrate to give crude 3-(4-chlorophenyl)-4-(4-difluoromethoxyphenyl)-2-pyrazoline (Intermediate C). This was suspended in dry ether and treated with 4-chlorophenyl isocyanate together with a few drops of triethylamine and the product worked up in conventional manner whereby there was obtained *N*,3-bis(4-chlorophenyl)-4-(4-difluoromethoxyphenyl)-2-pyrazoline-1-carboxamide, m.p. 135—7°C (Compound 1).

In a similar manner the following products were obtained.

Compounds containing a methylsulphonyl group were obtained by oxidising the corresponding compound containing a methylthio group using aqueous hydrogen peroxide in acetic acid.

Compounds in which $R^1$ is methyl were obtained by treating the corresponding compound where $R^1$ is hydrogen with methyl iodide in the presence of sodium hydride and dimethyl formamide (in the case of sulphonyloxy containing compounds) or in the presence of potassium hydroxide and acetone. Compounds in which $R^1$ is allyl were obtained from the corresponding compound where $R^1$ is hydrogen by reaction with allyl bromide in the presence of potassium hydroxide.

The position of the $R^4$ containing phenyl is given in the column headed A.

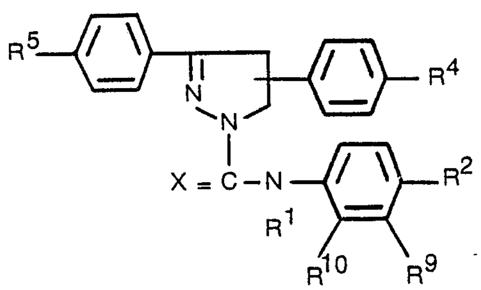

| Compound No. | X | $R^5$ | $R^1$ | $R^4$ | A | $R^9$ | $R^2$ | $R^{10}$ | m.p. (°C) |
|---|---|---|---|---|---|---|---|---|---|
| 2 | O | Cl | H | $CHF_2O$ | 4 | H | F | H | 141—3 |
| 3 | O | Cl | H | $CHF_2O$ | 4 | H | Br | H | 148—50 |
| 4 | O | Cl | H | $CHF_2O$ | 4 | Cl | H | H | 137—9 |
| 5 | O | Me | H | $CHF_2O$ | 4 | H | F | H | 139—40 |
| 6 | O | Me | H | $CHF_2O$ | 4 | H | Cl | H | 140—2 |
| 7 | O | Me | H | $CHF_2O$ | 4 | H | Br | H | 136—8 |
| 8 | O | $CHF_2O$ | H | $CHF_2O$ | 4 | H | Cl | H | 101—4 |
| 9 | O | H | H | $CHF_2O$ | 4 | H | Cl | H | 136—9 |
| 10 | O | F | H | $CHF_2O$ | 4 | H | Cl | H | 114—6 |
| 11 | O | MeS | H | $CHF_2O$ | 4 | H | Br | H | 110—3 |
| 12 | O | H | H | $CHF_2O$ | 4 | H | Br | H | 136—9 |
| 13 | O | MeS | H | $CHF_2O$ | 4 | H | Cl | H | 112—3 |
| 14 | S | Cl | H | $CHF_2O$ | 4 | H | Cl | H | 175—7 |
| 15 | O | Cl | H | $CHF_2O$ | 4 | H | Me | H | 180—3 |
| 16 | O | Cl | H | $CHF_2O$ | 4 | H | $NO_2$ | H | 191—4 |
| 17 | O | F | H | $CHF_2O$ | 4 | $CF_3$ | H | H | 140—1 |
| 18 | O | F | H | $CHF_2O$ | 4 | H | Br | H | 140—2 |
| 19 | O | F | H | $CHF_2O$ | 4 | Cl | Cl | H | 130—3 |
| 20 | O | Cl | H | $CHF_2O$ | 4 | $—OCH_2O—$ | | H | 187—9.5 |
| 21 | O | Cl | H | $CHF_2O$ | 4 | H | MeS | H | 154—6 |

| Compound No. | X | $R^5$ | $R^1$ | $R^4$ | A | $R^9$ | $R^2$ | $R^{10}$ | m.p. (°C) |
|---|---|---|---|---|---|---|---|---|---|
| 22 | O | Cl | H | $CHF_2O$ | 4 | H | CN | H | 179—82 |
| 23 | O | Cl | H | $CHF_2O$ | 4 | H | $CF_3$ | H | 135—7 |
| 24 | O | Cl | H | $CHF_2O$ | 4 | H | $COOPr^i$ | H | 163—6 |
| 25 | O | Br | H | $CHF_2O$ | 4 | H | $CF_3$ | H | 134—5 |
| 26 | O | Br | H | $CHF_2O$ | 4 | H | Cl | H | 141—4 |
| 27 | O | Br | H | $CHF_2O$ | 4 | H | $COOPr^i$ | H | 163—5 |
| 28 | O | Cl | H | $CHF_2O$ | 4 | $CF_3$ | H | H | 137—40 |
| 29 | O | H | H | $CHF_2O$ | 4 | H | F | H | 126—8 |
| 30 | O | H | H | $CHF_2O$ | 4 | H | $CF_3$ | H | 123—5 |
| 31 | O | H | Me | $CHF_2O$ | 4 | H | $CF_3$ | H | 129—31 |
| 32 | O | H | H | $CHF_2O$ | 4 | H | $Pr^i$ | H | 112—4 |
| 33 | O | H | H | $CHF_2O$ | 4 | H | $COOPr^i$ | H | 142.5—5 |
| 34 | O | Cl | H | $CHF_2O$ | 4 | H | $Pr^i$ | H | 129—32 |
| 35 | O | Cl | H | $CHF_2O$ | 4 | H | $EtSO_2O$ | H | 178—80 |
| 36 | O | H | Me | $CHF_2O$ | 4 | H | Br | H | 109—10 |
| 37 | O | Cl | H | $CHF_2O$ | 4 | H | Cl | Me | 141—3 |
| 38 | O | Cl | Me | $CHF_2O$ | 4 | H | Cl | H | 119—20 |

| Compound No. | X | $R^5$ | $R^1$ | $R^4$ | A | $R^9$ | $R^2$ | $R^{10}$ | m.p. (°C) |
|---|---|---|---|---|---|---|---|---|---|
| 39 | O | Cl | H | $CF_2HCF_2O$ | 5 | H | $Pr^i$ | H | 171—3 |
| 40 | O | Cl | H | $CF_2HCF_2O$ | 5 | H | $COOPr^i$ | H | 146—3 |
| 41 | O | Cl | H | $CF_2HCF_2O$ | 5 | H | F | H | 172—4 |
| 42 | O | H | H | $CHF_2O$ | 4 | H | $COCH_3$ | H | 183—4 |
| 43 | O | Cl | H | $CF_2HCF_2O$ | 5 | H | SMe | H | 186—90 |
| 44 | O | Cl | H | $CF_2HCF_2O$ | 5 | $—OCH_2O—$ | | H | 165—7.5 |
| 45 | O | Cl | H | $CF_2HCF_2O$ | 5 | H | CN | H | 225—6 |
| 46 | O | H | H | $CHF_2O$ | 4 | H | Me | H | 158—61 |
| 47 | O | Cl | H | $CF_2HCF_2O$ | 5 | H | Cl | H | 166—8 |
| 48 | O | $MeSO_2$ | H | $CHF_2O$ | 4 | H | Cl | H | 205—7 |
| 49 | O | Cl | H | $CHF_2O$ | 5 | H | $Pr^i$ | H | 144—6 |
| 50 | S | Cl | H | $CHF_2O$ | 5 | H | Cl | H | 173—6 |
| 51 | O | Cl | H | $CHF_2O$ | 5 | H | F | H | 168—71 |
| 52 | O | Cl | H | $CHF_2O$ | 5 | H | Cl | H | 216—7 |
| 53 | O | H | H | $CHF_2O$ | 4 | H | $EtSO_2O$ | H | 153—5 |
| 54 | O | H | H | $CHF_2O$ | 4 | H | Cl | Me | 177—9 |
| 55 | O | Cl | allyl | $CHF_2O$ | 4 | H | Cl | H | oil |
| 56 | O | Cl | H | $CHF_2O$ | 4 | H | $CHF_2O$ | H | 127—30 |
| 57 | O | H | H | $CHF_2O$ | 4 | H | $CF_2HCF_2O$ | H | 160—1 |
| 58 | | H | H | $CHF_2O$ | 4 | H | $CF_2BrO$ | H | 140—2 |

| Compound No. | X | $R^5$ | $R^1$ | $R^4$ | A | $R^9$ | $R^2$ | $R^{10}$ | m.p. (°C) |
|---|---|---|---|---|---|---|---|---|---|
| 59 | O | Cl | H | $CHF_2O$ | 4 | H | $CF_2BrO$ | H | 175—6.5 |
| 60 | O | Cl | H | $CHF_2O$ | 4 | H | $CF_2HCF_2O$ | H | 179—80.5 |
| 61 | O | Cl | H | $CF_3CH_2O$ | 4 | H | Cl | H | 168.5—70 |

During the course of preparation of the above, various intermediates were prepared. In many cases they were not isolated but used in the next stage without purification, the structures having been confirmed by n.m.r. The physical properties of these intermediates of compounds where the $R^4$-containing phenyl is in the 4 position are as follows. Temperatures are melting points in °C.

| $R^5$ | $R^4$ | Type A | Type B | Type C |
|---|---|---|---|---|
| $CHF_2O$ | $CHF_2O$ | | oil | |
| F | $CHF_2O$ | | oil | |
| H | $CHF_2O$ | 88—92.5 | oil | oil |
| MeS | $CHF_2O$ | 130—3 | oil | |
| Br | $CHF_2O$ | | oil | |

Type A intermediates in which $R^5$ is $CF_3SO_2O$ were prepared using trifluoromethanesulphonic anhydride or fluoride, instead of the sulphonyl chloride. For those compounds in which the $R^4$ containing phenyl is in the 5 position the type B intermediate was obtained by reacting a 4-haloalkoxy benzaldehyde with a 4-substituted acetophenone in the presence of aqueous sodium hydroxide at room temperature.

In this way the following were obtained.

a) 1-(4-chlorophenyl)-3-[4-(1,1,2,2-tetrafluoroethoxy)phenyl]-2-propen-1-one, m.p. 100—2°C.

b) 1-(4-chlorophenyl)-3-(4-difluoromethoxyphenyl)-2-propen-1-one, m.p. 105—8°C.

The type C intermediates were obtained from these by reaction with hydrazine hydrate. Since the products were unstable they were used in the next stage without purification.

Example 2

Sodium hydride (3.4 g of an 80% dispersion in oil) was added portionwise to 1-(4-chlorophenyl)-2-(4-difluoromethoxyphenyl)ethanone (24.3 g) in tetrahydrofuran. The mixture was stirred for ½ hour at room temperature and isopropyl iodide (8.8 ml) added dropwise. The mixture was heated under reflux for 4 hours and then filtered and evaporated. The residue was added to ether (150 ml) and water (50 ml). The ether layer was dried and evaporated and the residue subjected to flash column chromatography to give 1-(4-chlorophenyl)-2-(4-difluoromethoxyphenyl)-4-methylbutanone, obtained as a yellow oil. Bromine (2.5 ml) was added dropwise to this (14.4 g) in carbon tetrachloride. The mixture was stirred at room temperature for 24 hours and then evaporated. Dimethylformamide (75 ml) and lithium chloride (8.7 g) were added portionwise and the mixture heated at 130°C for 3 hours. It was then cooled, poured into water and extracted with ether. The extract was dried and evaporated and the residue subjected to flash column chromatography to give 1-(4-chlorophenyl)-2-(4-difluoromethoxyphenyl)-4-methyl-2-buten-1-one, obtained as an orange oil. Hydrazine hydrate (15.2 ml of a 64% solution) was added and the mixture stirred under nitrogen and in the dark for 24 hours. The mixture was then evaporated, ether (200 ml) and water (200 ml) added and the ether layer dried and evaporated to give crude 3-(4-chlorophenyl)-4-(difluoromethoxyphenyl)-5,5-dimethyl-2-pyrazoline. This was then treated with the appropriate phenyl isocyanate as described in Example 1 to give the following

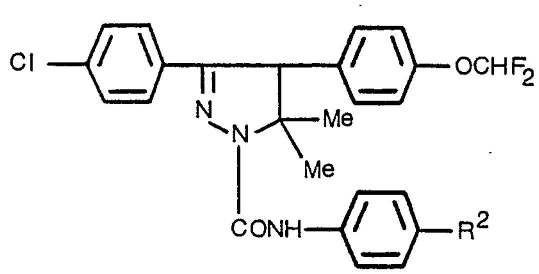

| Compound No | $R^2$ | mp (°C) |
|---|---|---|
| 201 | $SCH_3$ | 119—20 |
| 202 | $Pr^i$ | 138—40 |
| 203 | Cl | 144—5 |
| 204 | $COOPr^i$ | 135—7 |

Example 3

Aliquots of acetone solutions of test compounds at various concentrations were applied to 9 cm diameter filter papers placed in the bottom of 9 cm diameter glass dishes closed by glass lids.

The treated surfaces, together with controls treated with acetone alone, were then infested with adult houseflies, *Musca domestica*) and held at 25°C for 24 hours.

The percentage mortality of the insects was then recorded. Compounds 1, 2, 3, 6, 7, 9, 10, 13, 18, 19, 21, 23, 25, 26, 29, 31 and 36 have an $LD_{50}$ of less than 1000 mg/$m^2$ of filter paper.

Compounds 1 and 9 were compared with their isomers disclosed in EP 58424. The results were as follows. In all these compounds X, $R^1$, $R^9$, $R^2$ and $R^{10}$ are the same. The differences are that the groups at $R^4$ and $R^5$ are reversed.

| | Compound of Invention | | | Compound of EP 58424 | | |
|---|---|---|---|---|---|---|
| | $R^4$ | $R^5$ | $LD_{50}$ (mg/$m^2$) | $R^4$ | $R^5$ | $LD_{50}$ (mg/$m^2$) |
| 1 | $CHF_2O$ | Cl | 300—1000 | Cl | $CHF_2$ | >1000 |
| 9 | $CHF_2O$ | H | 100—300 | H | $CHF_2O$ | >1000 |

Example 4

Cabbage leaves were dipped in aqueous dispersions at various concentrations of test compound and the leaves allowed to dry. The dispersions contained 500 mg/litre of an ethylene oxide/nonylphenol wetting agent. Each leaf was placed in a petri dish with a lid and 10 second instar larvae of *Spodoptera littoralis* applied to each leaf. After three days untreated leaves are added to each replicate. Two days later an assessment was made of the mortality. Two replications were carried out at each concentration of each compound and an $LD_{50}$ calculated.

Compounds 1—23, 25, 26, 29, 32, 35, 36, 41 and 47 have an $LD_{50}$ of less than 300 mg/litre.

Example 5

Compounds were tested in a similar manner to Example 6 but using *Heliothis armiger* fed on tobacco leaves.

Compounds 1—4, 8—10, 13, 14, 18, 20—23, 26, 30 and 47 have an $LD_{50}$ of less than 300 mg/litre.

Example 6

Third instar larvae of the diamond-backed moth, *Plutella xylostella* were ingested onto cabbage leaves and 24 hours later the plant and larvae were sprayed with dispersions of compound as described in Example 3. After drying ingested leaves were placed in petri dishes. An assessment of mortality was made 5 days later.

Compounds 1—39 and 41—43 had an $LD_{50}$ of less than 1000 mg/litre.

Example 7

1 ml aliquots of an acetone solution containing test compound at various concentrations were applied to cotton wool dental rolls 1 cm x 2 cm, contained in glass vials 2 cm diameter x 5 cm long. After drying, the treated materials were then impregnated with 1 ml of nutrient solution, infested with third instar larvae of the Mediterranean fruit-fly, (*Ceratitis capitata)* or first instar larvae of sheep blow fly *Lucilia sericata*), closed by a cotton wool plug and held at 25°C for 24 hours.

The percentage mortality of the insects was then recorded. Compounds 1—3, 6 and 7 have an $LD_{50}$ of less than 100 mg/litre against *Ceratitis capitata* and compounds 8—32, 34—45 and 53—55 have an $LD_{50}$ of less than 1000 mg/litre against *Licilia sericata.*

In all the test Examples, less than 5% mortality was recorded for the controls.

Example 8

This example illustrates typical formulations using the compound of the invention.

| Wettable powder concentrate | % w/w |
|---|---|
| Compound No 1 | 25 |
| Sodium lignosulphonate | 5 |
| China clay | 70 |

Bait

| | % w/w |
|---|---|
| Wettable powder concentrate from above to give active ingredient content of | 0.4 |
| Apple pomice | 37.2 |
| Rolled bran | 37.2 |
| Plain white flour | to 100 |

Different compounds were also formulated in a similar manner and at various concentrations.

**Claims**

1. Compounds of formula I

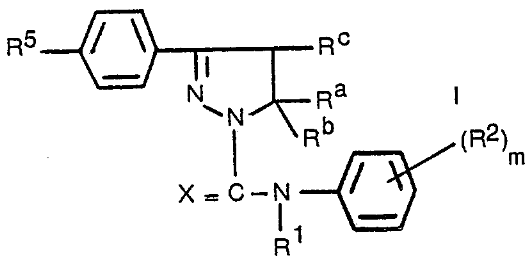

I

where $R^1$ is hydrogen, $C_{1-4}$-alkyl or allyl; X is oxygen or sulphur; each $R^2$, which may be the same or different, is halogen, $C_{1-4}$-alkyl, halo-$C_{1-4}$-alkyl, cyano, nitro, $C_{1-4}$-alkoxy, halo-$C_{1-4}$-alkoxy, $C_{1-4}$-alkylthio, halo-$C_{1-4}$-alkylthio, $C_{1-4}$-alkylsulphonyloxy, $C_{1-4}$-alkylsulphonyl, $C_{1-4}$-alkoxycarbonyl or $C_{1-4}$-alkanoyl or two adjacent $R^2$ groups together with the carbons to which they are attached form a 5 to 7 membered ring which may comprise one or more oxygen atoms; m is 0 to 4; either

a) $R^a$ is hydrogen and one of $R^b$ and $R^c$ is phenyl, para substituted by the group $R^4$, and the other is hydrogen; or

b) $R^a$ and $R^b$ are both $C_{1-4}$-alkyl and $R^c$ is phenyl para substituted by the group $R^4$; where $R^4$ is halo-$C_{1-4}$-alkoxy; $R^5$ is $R^8SO_2O$, hydrogen, halogen, $C_{1-4}$-alkyl, halo-$C_{1-4}$-alkoxy, $C_{1-4}$-alkylthio or $C_{1-4}$-alkylsulphonyl, wherein $R^8$ is $C_{1-4}$-alkyl or halo-$C_{1-4}$-alkyl.

2. Compounds according to claim 1 in which $R^a$ and $R^b$ are hydrogen.

3. Compounds according to claim 1 or 2 in which $R^5$ is hydrogen or halogen.

4. Compounds according to claim 1, 2 or 3 in which $R^4$ is difluoromethoxy.

5. Compounds according to claim 1 in which m is 1 and $R^2$ is in the para position.

6. Compounds according to claim 5 in which $R^2$ is chlorine, fluorine, bromine, methylthio or trifluoromethyl.

7. Compounds according to any one of the preceding claims in which $R^1$ is hydrogen.

8. *N*,3-Bis(4-chlorophenyl)-4-(4-difluoromethoxyphenyl)-2-pyrazoline-1-carboxamide.

9. 3-(4-chlorophenyl)-4-(4-difluoromethoxyphenyl)-*N*-(4-trifluoromethylphenyl)-2-pyrazoline-1-carboxamide.

10. 4-(4-Difluoromethoxyphenyl)-3-phenyl-*N*-(4-trifluoromethylphenyl)-2-pyrazoline-1-carboxamide.

11. An insecticidal composition comprising a compound as claimed in any one of the preceding claims in association with an agronomically acceptable carrier.

12. A method of combating insects which comprises applying to the insects or their locus a compound claimed in any one of claims 1 to 10.

13. Compounds of formula II

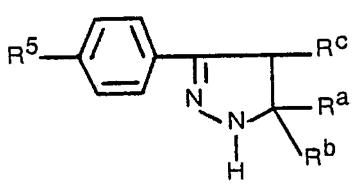

II

in which $R^a$, $R^b$, $R^c$ and $R^5$ have the meanings given in claim 1.

**Patentansprüche**

1. Verbindungen der Formel I

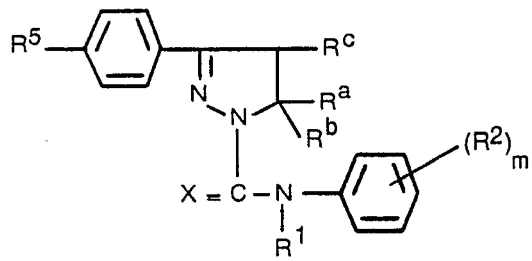

I

in der

$R^1$ Wasserstoff, $C_{1-4}$-Alkyl oder Allyl ist;

X Sauerstoff oder Schwefel ist;

$R^2$ gleich oder verschieden und ist Halogen, $C_{1-4}$-Alkyl, Halo-$C_{1-4}$-alkyl, Cyano, Nitro, $C_{1-4}$-Alkoxy, Halo-$C_{1-4}$-alkoxy, $C_{1-4}$-Alkylthio, Halo-$C_{1-4}$-alkylthio, $C_{1-4}$-Alkylsulfonyloxy, $C_{1-4}$-Alkylsulfonyl, $C_{1-4}$-Alkoxycarbonyl oder $C_{1-4}$-Alkanoyl ist oder zwei $R^2$ Gruppen bilden zusammen mit den benachbarten Kohlenstoffatomen einen 5- 7-gliedrigen Ring, der ein oder mehrere Sauerstoffatome enthalten kann;

m 0 bis 4 ist;

a) falls $R^a$ Wasserstoff ist, ist $R^b$ oder $R^c$ freies oder durch die Gruppe $R^4$ parasubstituiertes Phenyl bzw. Wasserstoff;

b) falls $R^a$ und $R^b$ gemeinsam $C_{1-4}$-Alkyl sind, ist $R^c$ durch die Gruppe $R^4$ parasubstituiertes Phenyl, wobei $R^4$ Halo-$C_{1-4}$-alkoxy bedeutet;

$R^5$ Wasserstoff, Halogen, $C_{1-4}$-Alkyl, Halo-$C_{1-4}$-alkoxy, $C_{1-4}$-Alkylthio, $C_{1-4}$-Alkylsulfonyl oder die Gruppe $R^8SO_2O$ ist, wobei $R^8$ $C_{1-4}$-Alkyl oder Halo-$C_{1-4}$-alkyl ist.

2. Verbindungen gemäß Anspruch 1, wobei $R^a$ und $R^b$ Wasserstoff ist.

3. Verbindungen gemäß Anspruch 1 oder 2, wobei $R^5$ Wasserstoff oder Halogen ist.

4. Verbindungen gemäß Anspruch 1, 2 oder 3, wobei $R^4$ Difluormethoxy ist.

5. Verbindungen gemäß Anspruch 1, wobei $m = 1$ und $R^2$ in para-Position ist.

6. Verbindungen gemäß Anspruch 5, wobei $R^2$ Chlor, Fluor, Brom, Methylthio oder Trifluormethyl ist.

7. Verbindungen gemäß den vorgenannten Ansprüchen, wobei $R^1$ Wasserstoff ist.

8. *N*,3-Bis(4-chlorphenyl)-4-(4-difluormethoxyphenyl)-2-pyrazolin-1-carboxamid.

9. 3-(4-Chlorphenyl)-4-(4-difluormethoxyphenyl)-*N*-(4-trifluormethylphenyl)-2-pyrazolin-1-carboxamid.

10. 4-(4-Difluormethoxyphenyl)-3-phenyl-*N*-(4-trifluormethylphenyl)-2-pyrazolin-1-carboxamid.

11. Insektizides Mittel, enthaltend eine Verbindung gemäß den vorgenannten Ansprüchen, in Mischung mit einem inerten Trägermaterial.

12. Methode zur Bekämpfung von Insekten, dadurch gekennzeichnet, daß man auf Insekten oder deren Lebensraum eine Verbindung gemäß den Ansprüchen 1 bis 10 einwirken läßt.

13. Verbindungen der Formel II

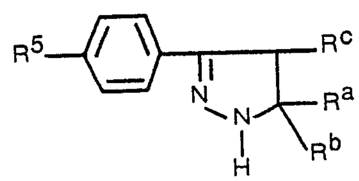

II

in welcher $R^a$, $R^b$, $R^c$ und $R^5$ die in Anspruch 1 angegebene Bedeutung haben.

**Revendications**

1. Composés de formule I:

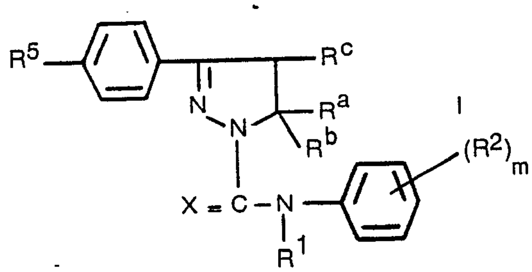

I

dans laquelle $R^1$ est un atome d'hydrogène ou un groupe allylique ou alkyle en $C_{1-4}$.

X est l'oxygène ou le soufre,

chaque groupe $R^2$, ces groupes pouvant être identiques ou différents, est un atome d'halogène, un groupe alkyle en $C_{1-4}$, un groupe haloalkylique en $C_{1-4}$, un groupe cyano, un groupe nitro, un groupe alcoxy en $C_{1-4}$, un groupe haloalcoxy en $C_{1-4}$, un groupe alkylthio en $C_{1-4}$, un groupe haloalkylthio en $C_{1-4}$, un groupe alkylsulfonyloxy en $C_{1-4}$, un groupe alkylsulfonyle en $C_{1-4}$, un groupe alcoxycarbonyle en $C_{1-4}$ ou un groupe alcanoyle en $C_{1-4}$, deux groupes $R^2$ adjacents pouvant aussi former, avec les atomes de carbone auxquels ils sont fixés, un noyau de cinq à sept atomes, pouvant contenir un ou plusieurs atomes d'oxygène,

m est compris entre 0 et 4, et soit

a) $R^a$ est un atome d'hydrogène et l'un des substituants $R^b$ et $R^c$ est un groupe phényle substitué en para par le groupe $R^4$, et l'autre est un atome d'hydrogène, ou

b) $R^a$ et $R^b$ sont tous deux des groupes alkyle en $C_{1-4}$ et $R^c$ est un groupe phényle substitué en para par le groupe $R^4$, le groupe $R^4$ étant un groupe haloalcoxy en $C_{1-4}$, $R^5$ est $R^8SO_2O$, un atome d'hydrogène ou d'halogène, un groupe alkyle en $C_{1-4}$, un groupe haloalcoxy en $C_{1-4}$, un groupe alkylthio en $C_{1-4}$ ou un groupe alkylsulfonyle en $C_{1-4}$, $R^8$ étant un groupe alkyle en $C_{1-4}$ ou un groupe haloalkyle en $C_{1-4}$.

2. Composés selon la revendication 1, dans lesquels $R^a$ et $R^b$ sont des atomes d'hydrogène.

3. Composés selon l'une des revendications 1 et 2, dans lequels $R^5$ est un atome d'hydrogène ou d'un halogène.

4. Composés selon l'une quelconque des revendications 1, 2 et 3, dans lequel $R^4$ est un groupe difluorométhoxy.

5. Composés selon la revendication 1, dans lesquels m est égal à 1 et $R^2$ est en position para.

6. Composés selon la revendication 5, dans lesquels $R^2$ est le chlore, le fluor, le brome, un groupe méthylthio ou un groupe trifluorométhyle.

7. Composés selon l'une quelconque des revendications précédentes, dans lesquels $R^1$ est un atome d'hydrogène.

8. N,3-Bis(4-chlorophényl)-4-(4-difluorométhoxyphenyl)-2-pyrazoline-1-carboxamide.

9. 3-(4-chlorophényl)-4-(4-difluorométhoxyphényl)-N-(4-trifluorométhylphényl)-2-pyrazoline-1-carboxamide.

10. 4-(4-Difluorométhoxyphényl)-3-phényl-N-(4-trifluorométhylphényl)-2-pyrazoline-1-carboxamide.

11. Composition insecticide comprenant un composé selon l'une quelconque des revendications précédentes, en association avec un véhicule acceptable au point de vue agronomique.

12. Procédé de lutte contre les insectes, qui comprend l'application, aux insects ou à leurs lieux d'habitation, d'un composé selon l'une quelconque des revendications 1 à 10.

13. Composés de formule II

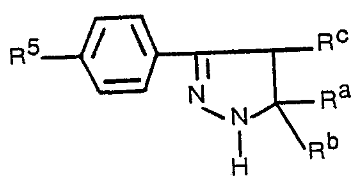

II

dans lesquels $R^a$, $R^b$, $R^c$ et $R^5$ ont les significations indiquées dans la revendication 1.